# EUROPEAN PATENT SPECIFICATION

(11) **EP 3 391 610 B1**
(45) Date of publication and mention of the grant of the patent: **20.10.2021**
(21) Application number: 16791634.5
(22) Date of filing: 09.11.2016
(51) Int. Cl.: H04L 29/06, H04L 9/08, G16H 40/67

(54) **METHOD AND SYSTEM FOR KEY DISTRIBUTION BETWEEN A SERVER AND A MEDICAL DEVICE**
VERFAHREN UND SYSTEM ZUR SCHLÜSSELVERTEILUNG ZWISCHEN EINEM SERVER UND EINER MEDIZINISCHEN VORRICHTUNG
PROCÉDÉ ET SYSTÈME POUR UNE DISTRIBUTION DE CLÉ ENTRE UN SERVEUR ET UN DISPOSITIF MÉDICAL

(30) Priority: 17.12.2015 WO PCT/EP2015/307034
(43) Date of publication of application: 24.10.2018
(73) Proprietor: Fresenius Vial SAS, 38590 Brézins (FR)
(72) Inventor: DERVYN, Olivier, 38210 Tullins Isère/R.A (FR)
(74) Representative: Fresenius Kabi Deutschland GmbH
(86) International application number: PCT/EP2016/077133
(87) International publication number: WO 2017/102183

(56) References cited:
- US-A1- 2004 098 581
- US-A1- 2005 204 134
- US-A1- 2006 218 397
- US-A1- 2011 179 405

## Description

The invention relates to a method and a system for key distribution between a server and a medical device.

The medical device may in particular be an infusion device, such as a volumetric or syringe infusion pump located for example on a rack at the bedside of a patient. The server and the medical device may for example be located within a hospital environment and may be configured to communicate with each other via a communication network, for example making use of the Internet Protocol (IP).

During setup or during operation of the medical device, for example the infusion device, messages are exchanged between the server and the medical device. These messages may comprise operational data as well as configuration data, which shall be protected from an access from the outside and from a manipulation by a non-authenticated third-party.

For the protection of data, typically, encryption technologies are used making use of complex cryptographic (and power consuming) algorithms (such as HTTPS). Medical devices, such as infusion pumps, due to limited CPU and memory capabilities however may not have the necessary computational power to use such complex encryption algorithms such that a security key distribution between a server and a medical device such as an infusion pump requires a different solution.

Commonly, symmetric-key algorithms and asymmetric-key algorithms are known and used for the encryption.

Symmetric-key algorithms use the same cryptographic keys for both encryption of plaintext and decryption of ciphertext. The keys may be identical, or there may be a simple transformation to go between the two keys. In practice the key is called a "shared secret" between two or more parties. The main drawback of symmetric key encryption is the key distribution vulnerability. If anybody has access to the secret key (and algorithm to cipher data), he has access to the secret data.

Asymmetric-key algorithms (public-key cryptography) refer to a set of cryptographic algorithms based on non-reversible mathematical properties. Asymmetric-key algorithms, unlike symmetric-key algorithms, do not require a secure channel for the initial exchange of one (or more) secret keys between the parties. Asymmetric-key algorithms are fundamental security ingredients in security applications and protocols (for example Transport Layer Security (TLS) or PGP).

US2005204134 A1 discloses a system and a method for securely authenticating a data exchange session with an implantable medical device. US2011179405 A1 discloses methods and systems configured to receive medical device data transmitted in any format and from any medical device. US2006218397 A1 discloses a secure communication between a server and a client by using a key obtained from an intermediate device.

It is an object of the invention to provide a method and a system for the security key distribution between a server and a medical device, the method in the system being suitable even for medical devices having low computational and memory capabilities. This object is achieved by means of a method according to the features of claim 1.

The invention addresses security key management and broadcast for medical devices such as infusion pumps. The invention enables to distribute and manage security keys between a server and a medical device for a secure communication between the server and the medical device. By means of the security key distribution an encrypted communication as well as an authentication between the medical device and the server becomes possible.

A solution hence is proposed to solve confidentiality, integrity and authentication of both parts during security key distribution. Key distribution is a method to distribute cryptographic keys between two (or more) parties, allowing use of a cryptographic algorithm. A fundamental problem of key distribution is how to exchange keys (or other needed information) such that no one else can obtain a copy. The invention proposes a solution to get advantage of public key cipher algorithms (high level of security) to distribute keys and to get advantage of private key cipher algorithms (simplicity and low computing cost) to encrypt or decrypt messages.

One or multiple security keys may be distributed from the server to one or multiple medical devices such as infusion pumps. In particular, a key pair may be distributed from the server to a particular medical device in order to allow for an authentication and for an encrypted communication between the server and the medical device.

In another embodiment, a security key for example for a WiFi connectivity of the medical device may be distributed from the server to the medical device, for example a WPA2 password or a Radius Certificate.

The computing device may for example be a personal computer (PC) such as a laptop computer. The computing device however may also be a mobile device such as a mobile phone, a tablet computer or another mobile communication device having sufficient computational capabilities.

The first communication link via which the server is connected to the computing device may for example be a link making use of the Internet protocol. The link may for example make use of an HTTPS session and hence is protected by means of cryptographic algorithms implemented in HTTPS. The computing device hence communicates with the server via a preferably secured communication link such that the security key may be sent from the server to the computing device via a secured link, in particular in the context of an HTTPS session.

The second communication link between the computing device and the medical device may in particular be a wired link, such as a serial link (for example a RS232 link). The computing device hence locally is connected to the medical device via a dedicated communication line such that no particular and computationally expensive security algorithms are required to protect a communication between the computing device and the medical device.

The security key is provided at the server and is sent, via the first communication link, from the server to the computing device. The computing device may have sufficient computational power such that complex, high level cryptographic algorithms, for example in the context of an HTTPS session, may be used to protect the communication between the server and the computing device when transmitting the security key from the server to the computing device.

The computing device then sends the security key received from the server on to the medical device via the second communication link, wherein for this no encryption is necessary if a local link such as a wired link, in particular a serial link, is established between the computing device and the medical device. Hence, the medical device does not require extensive computational power, because complex cryptographic algorithms are not necessary to protect the communication between the computing device and the medical device.

In one embodiment, the computing device uses a dedicated software denoted as security application for distributing the security key from the server to the medical device. The dedicated software of the security application in particular establishes the communication with the server and makes sure that the security key received from the server is not stored by the computing device, but is only sent on to the medical device and is subsequently deleted at the computing device.

The medical device, for example the infusion pump, is constituted such that the security key cannot be read when accessing the medical device.

In one embodiment, a key pair comprising a server key and a device key is provided by the server, is retrieved from the computing device and is sent by the computing device to the medical device. Herein, the server may generate a multiplicity of different, dedicated key pairs associated with a multiplicity of medical devices, for example different infusion devices. Each medical device hence is associated with a dedicated key pair having a specific server key and a specific device key, the server keys and device keys used by the different medical devices being different from each other.

For example, medical device A may have server key A and device key A, whereas medical device B may have server key B and device key B. The key pairs of the different medical devices hence are distinct from each other

For each medical device the key pair may be retrieved from the server by the computing device and may be transmitted to the particular medical device. Via the computing device the different key pairs hence are distributed to the different medical devices.

Once the key pairs are distributed to the different medical devices, a secure communication may take place between a medical device and the server, for example during operation of the medical device, such as an infusion pump, to provide operational data to the server. The communication link between the medical device and the server may for example make use of standard communication protocols, for example HTTP or any other kind of protocol (IP and non IP Protocol), wherein the key pair associated with a particular medical device may be used to authenticate the medical device at the server as well as to encrypt messages sent between the medical device and the server.

For example, for less critical data the medical device may send a clear message to the server within an HTTP session, the message having a signature generated according to at least one of the device key and the server key of the key pair associated with the medical device. The signature can be read by the server, and by means of the signature the server can authenticate the medical device such that it is ensured that the message is received from a trusted medical device and has not been manipulated by a third-party. In particular, if the method would be manipulated on its way from the medical device to the server, the signature would be changed, which could be detected by the server. If the server receives and detects a correct signature, the server knows that the message has not been manipulated and has been sent by an authenticated medical device.

To avoid message replay from a non-trusted third-party, each message may have a non-static part (for example date and time) to be sure to obtain a different signature computation for each message (because the same message with the same key may otherwise provide the same signature). For this, a time stamp or a counter may be added in the message body, and the server and/or the medical device may be configured to reject a message with a bad time stamp (i.e., a time stamp which is too old) or with a bad message counter. For these reasons, a time synchronization between each item of the system (medical device, server and computing device) may be initially performed.

In another example, the communication between the medical device and server may be encrypted, in particular for data that is to be protected. For encryption, at least one of the device key and the server key of the key pair may be used, wherein the medical device encrypts the message according to the key pair, and the server decrypts the message received from the medical device making use of the key pair, and vice versa. Again, to avoid a message replay from a non-trusted third-party, each message may have a non-static part (for example date and time) to be sure to obtain a different encryption for each message (because the same message with the same key may otherwise provide the same encryption).

It may be desirable to change a security key associated with a medical device after some time of usage, for example after sending a predefined amount of messages between the server and the medical device. A change of the security key may be requested by the medical device by sending an encrypted message to the server, upon which the server sends the new security key, for example a new key pair, in an encrypted message to the medical device. The message in which the new security key is sent to the medical device herein is encrypted making use of the old security key, for example the old key pair. Upon receiving the new security key, for example the new key pair, at the medical device, for the further communication between the medical device and the server then the new security key is used.

A specific change key message could be sent to the server to advise it of a key change. In this case, the message is encrypted with the old keys.

The object is also achieved by a system for key distribution between a server and a medical device according to the features of claim 10.

The advantages and advantageous embodiments described above for the method equally apply also to the system, such that it shall be referred to the above.

The idea underlying the invention shall subsequently be described in more detail with regard to the embodiments shown in the figures. Herein,
- Fig. 1: shows a system comprising a server, a computing device and a multiplicity of medical devices;
- Fig. 2: shows a communication between a medical device and the server using a message comprising a signature for authentication; and
- Fig. 3: shows a communication between a medical device and the server using an encrypted message.

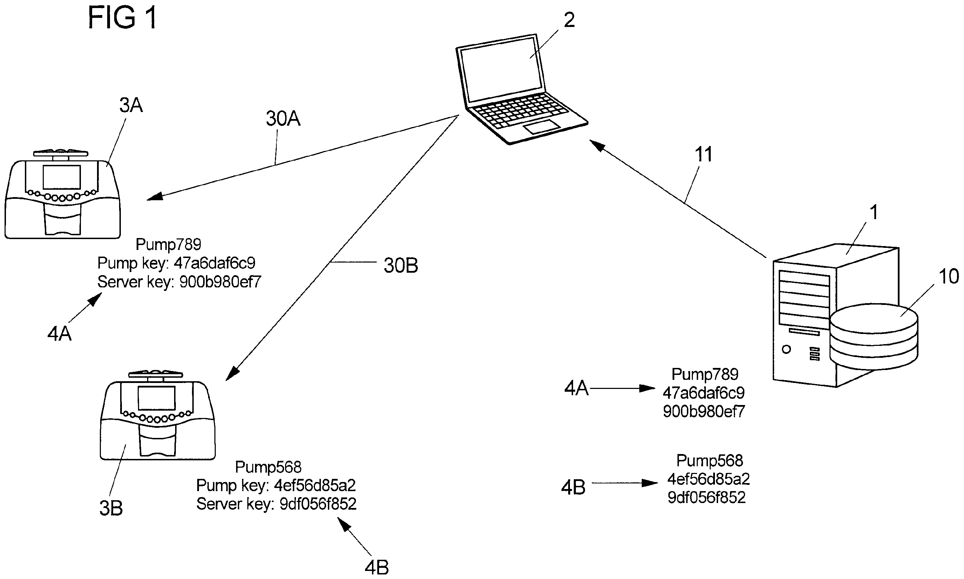

Fig. 1 shows a system comprising a server 1, a computing device 2 such as a portable laptop computer and a multiplicity of medical devices 3A, 3B in the shape of infusion pumps. The system may be located in a hospital environment, wherein the server 1 may for example be placed at a central location in a hospital, and the infusion devices 3A, 3B may be placed on different wards in different rooms of the hospital.

The computing device 2 may be a portable device such that it for example can locally be connected to a particular medical device 3A, 3B.

In order to allow for a secure communication between the server 1 and the medical devices 3A, 3B, security keys in the shape of dedicated key pairs 4A, 4B are distributed from the server 1 via the computing device 2 to the different medical devices 3A, 3B. The key pairs 4A, 4B herein are generated at the server 1 and are stored in a security key database in a storage 10 of the server 1.

As indicated in Fig. 1, the server 1 generates, for each medical device 3A, 3B, a dedicated key pair 4A, 4B. Each key pair 4A, 4B comprises a server key and a device key, the server keys and the device keys being different between the different medical devices 3A, 3B.

In order to allow for a secure communication between the medical devices 3A, 3B and the server 1, the key pairs 4A, 4B generated at the server 1 must be distributed to the different medical devices 3A, 3B. This takes place via the computing device 2.

For distribution of the key pairs 4A, 4B, the computing device 2 establishes a communication link 11 with the server 1 and retrieves the key pairs 4A, 4B from the server 1. The computing device 2 is connected to the different medical devices 3A, 3B (at the same time or one after the other) via communication links 30A, 30B, which may for example be wired links such as serial links (e.g., RS 232 links). Via the local communication links 30A, 30B the key pairs 4A, 4B are sent to the medical devices 3A, 3B, such that a first medical device 3A receives a first key pair 4A, and a second medical device 3B receives a second key pair 4B and so on.

The distribution of the key pairs 4A, 4B from the server 1 to the medical devices 3A, 3B may take place via a security application running on the computing device 2. The security application can for example establish the communication link 11 to the server 1 and can transmit the received key pairs 4A, 4B to the medical devices 3A, 3B, without locally storing the key pairs 4A, 4B at the computing device 2. In this way it is made sure that the key pairs 4A, 4B do not become publicly known if for example the computing device 2 is lost or is attacked from the outside.

The server 1 may be set up such that only a specific security application running on a computing device 2 may receive the key pairs 4A, 4B. The key pairs 4A, 4B hence cannot be accessed by another device or another application from the outside.

The key pairs 4A, 4B are sent from the server 1 to the computing device 2 via a secured link making use of cryptographic algorithms, such as an asymmetric cryptographic algorithm, e.g. public key methods such as Transport Level Security (TLS) or PGP. Hence, the communication between the server 1 and the computing device 2 is protected.

Because the computing device 2 is locally connected via for example a wired link 30A, 30B to the medical devices 3A, 3B, the key pairs 4A, 4B can be send by the computing device 2 to the medical devices 3A, 3B in clear messages without using encryption. The medical devices 3A, 3B hence do not require substantial computational power and do not need to run complex cryptographic algorithms.

Upon distribution of the key pairs 4A, 4B, the communication between a medical device 3A, 3B and the server 1, for example for configuration of the medical device 3A, 3B or during operation of the medical device 3A, 3B, may take place in a secure fashion.

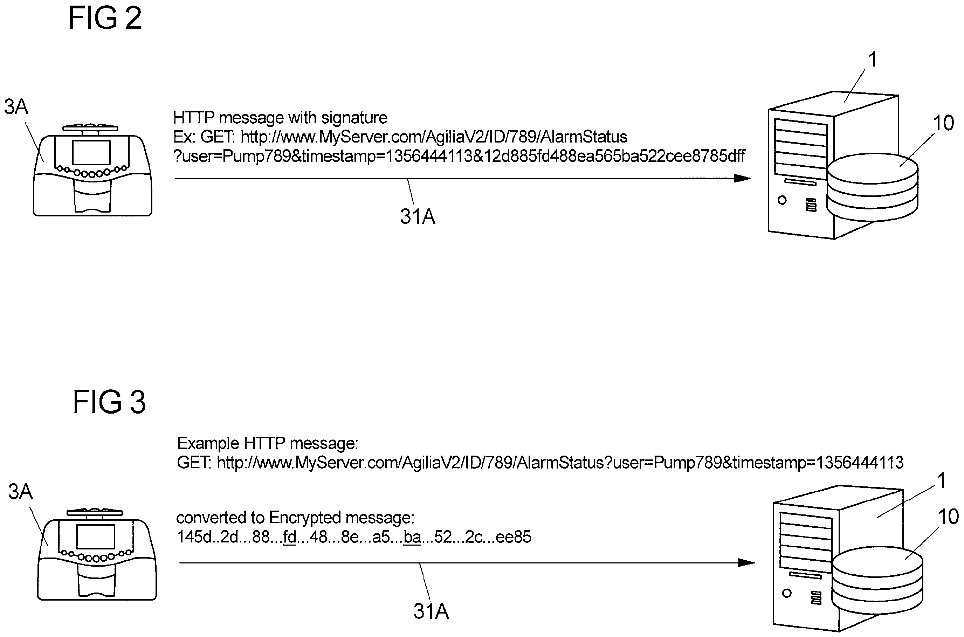

In particular, as illustrated in Fig. 2, a medical device 3A may send data in a clear message (for example within an HTTP session) comprising a signature generated according to the key pair 4A associated with the medical device 3A. The server 1 can read the signature and, according to the signature and the key pair 4A known to the server 1, can verify that the message has been sent by the medical device 3A (and not by another device) and has not been manipulated by another party. By means of the signature, hence, the medical device 3A is authenticated.

The signature may for example be generated by the medical device 3A using its device key. Upon receiving a message including the signature, the server 1 verifies the received signature according to the device key associated with the medical device 3A known to the server 1 by computing the signature and by comparing the computed signature to the received signature. Vice versa, the server 1 may send a clear message to the medical device 3A including a signature generated according to the server key, and the medical device 3A may verify the signature according to the server key known to the medical device 3A by computing the signature and comparing it to the received signature.

The message may include a timestamp or a counter and may hence have a non-static part. In this way it can be made sure that each message generated will have a different signature computed according to the key pair 4A. in particular, a message sent at a different time will have a different signature. In this way it can be made sure that a message replayed from a non-trusted third-party would have a non-matching signature, which can be detected by the server 1 respectively the medical device 3A.

Furthermore, as illustrated in Fig. 3, the medical device 3A may send an encrypted message (for example within an HTTPS session) being encrypted by the key pair 4A associated with the medical device 3A. The encrypted message is received by the server 1 and can be decrypted by the server 1 making use of the key pair 4A associated with the sending medical device 3A.

Again, the encryption done by the medical device 3A may make use of the device key associated with the medical device 3A. Upon receiving the encrypted message the server 1 can decrypt the message making use of the device key known to the server 1. Vice versa, the server 1 may send a message encrypted using the server key, and the medical device 3A may decrypt the received message using the server key known to the medical device 3A.

Again, a timestamp or counter may be included in the message such that the message contains a non-static part. Hence, a replay of the message from a non-trusted third-party is avoided, because in encryption of the message having another timestamp or another counter leads to a different encryption, which can be detected by the server 1 respectively the medical device 3A.

With the proposed scheme the distribution of the key pairs 4A, 4B from the server 1 to the medical devices 3A, 3B takes place in a secured fashion using highly secure cryptographic algorithms such as asymmetric key algorithms (public key cryptography), which generally are computationally expensive. This however does not pose a problem since the key distribution takes place via the computing device 2 running a security application. After key distribution, then, the further communication between the medical device 3A, 3B and the server 1, to exchange data between the medical device 3A, 3B and the server 1, may take place using the exchanged security key 4A, 4B. For this communication a symmetric key algorithm using a shared secret key may be used, which is computationally less expensive and hence does not pose a problem to a medical device 3A, 3B potentially having a reduced computing power.

The proposed scheme hence may make use of highly secure asymmetric key algorithms (public key algorithms) for the distribution of security keys. For the actual data exchange and communication, then, it may be made use of symmetric key algorithms (private key algorithms) which are computationally less expensive. Hence, the exchange of keys may make use of a different cryptographic algorithm than the later data communication.

Messages exchanged between a medical device 3A, 3B and the server 1 hence may be signed or encrypted using a secret key shared between the medical device 3A, 3B and the server 1. Each medical device 3A, 3B herein stores its secret key and the server key, and the server 1 stores the device key and its server key.

Each medical device 3A, 3B herein is associated with a dedicated key pair 4A, 4B having a specific device key and a specific server key. Because every medical device 3A, 3B is associated with a dedicated key pair 4A, 4B, a compromising of the server key in case of a device attack can be avoided.

The key pairs 4A, 4B may for example be distributed during the device configuration (via a security application running on the computing device 2).

Because the computing device 2 transfers the key pair 4A, 4B associated with a particular medical device 3A, 3B via a local link 30A, 30B, which in particular may be a wired link, for example a serial (RS232) link, to the particular medical device 3A, 3B, no particular encryption needs to be used for transferring the key pair 4A, 4B from the computing device 2 to the specific medical device 3A, 3B. Hence, no computationally expensive cryptographic algorithm must run on the medical devices 3A, 3B for the sake of distributing the key pairs 4A, 4B.

Generally, the key pairs 4A, 4B generated by the server 1 should be compliant with signature and/or encryption algorithms such as, for example, the SHA-1, AES-128 or AES-256 algorithm.

The secret key pair 4A, 4B associated with each medical device 3A, 3B should be stored in in the security server database of the storage 10 of the server 1 in encrypted form. For this, the server 1 should use an internal secret key to encrypt ("salt") all security data, in particular the key pairs 4A, 4B, in its database in the storage 10.

The server 1 should support a signature and/or encryption algorithm compliant with the signature and/or encryption algorithm used by the medical device 3A, 3B, for example the SHA-1, AES-128 or AES-256 algorithm.

Encryption is not required on the most part of the use cases. It is recommended to use encryption only optionally on the side of the medical device 3A, 3B in order to save computational power and energy of the medical device 3A, 3B (which may for example run using a battery). Hence, it may be chosen on the side of the medical device 3A, 3B whether a message is sent using a signature created according to the key pair 4A, 4B associated with the medical device 3A, 3B, or whether a message is encrypted using a private key algorithm making use of the key pair 4A, 4B associated with the medical device 3A, 3B.

Generally, the key pair 4A, 4B associated with a medical device 3A, 3B should be exchanged and replaced by a new key pair 4A, 4B on a frequent basis. The frequency of key replacement herein may depend on a desired security level for the particular medical device 3A, 3B. The change of a key pair 4A, 4B may for example be triggered by the medical device 3A, 3B by sending a request message to the server 1. This message can be encrypted by using the old key pair 4A, 4B by the medical device 3A, 3B, and can be decrypted by the server 1 using the old key pair 4A, 4B known to the server 1. The server 1 may then respond by sending a message to the medical device 4A, 4B including a new key pair 4A, 4B, wherein this message again may be encrypted using the old key pair 4A, 4B and may be decrypted by the medical device 3A, 3B using the old key pair 4A, 4B. For future messages, then, the new key pair 4A, 4B is used.

The server 1 should be set up to be fully secure and protected against cyber-attack and local attack (similar to, e.g., a RADIUS (short for Remote Authentication Dial-In User Service) server in a public key infrastructure). All key pairs 4A, 4B should be stored in a secret key container in the database of storage 10, possibly storing a device unique identifier, the server key and the device key of each key pair 4A, 4B in cipher form.

The server 1 may, in one embodiment, use a public/private key pair to be used for example in an AES-256 algorithm to encrypt other data. Public/private key pairs should also be stored in the secret key container of the database of storage 10.

In the security application of the computing device 2, authentication may for example be managed using a trusted authentication system based on, e.g., a X.509 certificate. A certificate herein may be linked to a user to authenticate the user on the security application.

The security application of the computing device 2 may, in one embodiment, be set up to authenticate the server 1 (to avoid identity theft) and should be protected by requiring a user to provide a login name and a password (managed for example by a so-called Lightweight Directory Access Protocol (LDAP)). The security application should use an authentication involving a certificate (or any other authentication technology) to prove the user identity of the user logged on to the security application to the server 1.

The security application of the computing device 2, upon connection to the medical device 3A, 3B via a local link, for example an RS232 serial link, may be set up to retrieve a unique ID from the medical device 3A, 3B, and may be set up to send the unique ID to the server 1 in order to request a secret key pair 4A, 4B from the server 1. The security application herein may add information to the request.

Each time the security application requests a key pair 4A, 4B from the server 1, a new key pair 4A, 4B should be generated.

The medical device 3A, 3B may for example be an infusion device such as a syringe infusion pump or a volumetric (peristaltic) infusion pump. The medical device 3A, 3B should have a unique ID for its own authentication. The medical device 3A, 3B should be able to receive secret keys from the security application of the computing device 2, which for example may be a laptop computer, a mobile device such as a smart phone or another computing device having sufficient computing power. The medical device 3A, 3B should store all secret key information received via the security application of the computing device 2 in a memory part protected against an external read operation. In particular, no maintenance or operating application of the medical device 3A, 3B should have access to the secret key information stored on the medical device 3A, 3B.

In case the distribution of a key pair 4A, 4B to a medical device 3A, 3B fails, or in case a problem with a key pair 4A, 4B arises, a new key pair 4A, 4B must be distributed to the medical device 3A, 3B. A restoration of a key pair 4A, 4B should be avoided.

The invention is not limited to the embodiments described above, but may be implemented in an entirely different fashion.

For example, a similar approach as the one described above for the distribution of key pairs for the communication between a medical device and a server may also be applied for configuring a medical device, for example an infusion device such as an infusion pump, to provide for a WiFi connectivity, for example for distributing a WPA2 password or a RADIUS Certificate to the medical device.

### List of Reference Numerals

- 1: Security server
- 10: Storage
- 11: Link
- 2: Computing device (PC)
- 3A, 3B: Medical device (infusion pump)
- 30A, 30B: Link
- 31A: Link
- 4A, 4B: Key pair

## Claims

1. Method for key distribution between a server (1) and a medical device (3A, 3B), wherein the medical device is an infusion device, comprising:
- providing, at the server (1), a security key (4A, 4B) in the shape of a dedicated key pair (4A, 4B) to be used for a secure data communication of the medical device (3A, 3B), wherein the server (1) provides the dedicated key pair (4A, 4B) comprising a server key and a device key,
- establishing a first communication link (11) between the server (1) and a computing device (2), wherein the first communication link is a secured link making use of an asymmetric key algorithm,
- establishing a second communication link (30A, 30B) between the computing device (2) and the medical device (3A,3B),
- retrieving, by the computing device (2), the security key (4A, 4B) from the server (1) via the first communication link (11), and
- transmitting, by the computing device (2), the retrieved security key (4A, 4B) to the medical device (3A, 3B) via the second communication link (30A, 30B), wherein the security key (4A, 4B) is not stored by the computing device (2), but is only transmitted to the medical device (3A, 3B), and
- upon distribution of the dedicated key pair (4A, 4B), establishing a third communication link (31A) between the medical device (3A, 3B) and the server (1) to transmit messages in between the medical device (3A, 3B) and the server (1) using the security key (4A, 4B), wherein the third communication link (31A) uses a symmetric key algorithm and wherein a message is encrypted using at least one of the device key and the server key of the key pair (4A, 4B).

2. Method according to claim 1, wherein the first communication link (11) is an Internet Protocol link.

3. Method according to claim 1 or 2, wherein the first communication link (11) uses an HTTPS session.

4. Method according to one of claims 1 to 3, wherein the second communication link (30A, 30B) is wired link.

5. Method according to one of the preceding claims, wherein the second communication link (30A, 30B) is a serial connection.

6. Method according to one of the preceding claims, wherein the server (1) generates a multiplicity of different, dedicated key pairs (4a, 4B) associated with a multiplicity of medical devices (3A, 3B), each key pair (4A, 4B) comprising a dedicated server key and a dedicated device key.

7. Method according to claim 6, wherein, for each medical device (3A, 3B), the associated dedicated key pair (4A, 4B) is retrieved from the server (1) by the computing device (2) and is transmitted to the medical device (3A, 3B).

8. Method according to one of the preceding claims, wherein a message comprises a signature generated using at least one of the device key and the server key of the key pair (4A, 4B) associated with the medical device (3A, 3B) and the server (1).

9. Method according to one of the preceding claims, wherein the medical device (1) requests, after initial distribution of the security key (4A, 4B), a new security key (4A, 4B) by sending a message to the server (1), and the server (1) sends the new security key (4A, 4B) in an encrypted message to the medical device (3A, 3B).

10. System for key distribution, the system comprising:
- a server (1) configured to provide a security key (4A, 4B) in the shape of a dedicated key pair (4A, 4B) to be used for a secure data communication of a medical device (3A, 3B), wherein the server (1) is configured to provide the dedicated key pair (4A, 4B) comprising a server key and a device key,
- a computing device (2), wherein the server (1) and the computing device (2) are configured to establish a first communication link (11) between the server (1) and the computing device (2), wherein the first communication link is a secured link making use of an asymmetric key algorithm,
- the medical device (3A, 3B), wherein the medical device (3A, 3B) is an infusion device, wherein the computing device (2) and the medical device (3A, 3B) are configured to establish a second communication link (30A, 30B) between the computing device (2) and the medical device (3A,3B),
wherein the computing device (2) is configured to retrieve the security key (4A, 4B) from the server (1) via the first communication link (11) and to transmit the retrieved security key (4A, 4B) to the medical device (3A, 3B) via the second communication link (30A, 30B), wherein the computing device (2) is further configured not to store the security key (4A, 4B), but to only transmit the security key (4A, 4B) to the medical device (3A, 3B),and wherein the medical device (3A, 3B) and the server (1) are configured to establish upon distribution of the dedicated key pair (4A, 4B) a third communication link (31A) between the medical device (3A, 3B) and the server (1) to transmit messages in between the medical device (3A, 3B) and the server (1) using the security key (4A, 4B), wherein the third communication link (31A) uses a symmetric key algorithm, and wherein a message is encrypted using at least one of the device key and the server key of the key pair (4A, 4B).

## Patentansprüche

1. Verfahren zur Schlüsselverteilung zwischen einem Server (1) und einer medizinischen Vorrichtung (3A, 3B), wobei die medizinische Vorrichtung eine Infusionsvorrichtung ist, umfassend:
- Bereitstellen, am Server (1), eines Sicherheitsschlüssels (4A, 4B) in Form eines dedizierten Schlüsselpaars (4A, 4B) zur Verwendung für eine sichere Datenkommunikation der medizinischen Vorrichtung (3A, 3B), wobei der Server (1) das dedizierte Schlüsselpaar (4A, 4B) bereitstellt, das einen Serverschlüssel und einen Vorrichtungsschlüssel umfasst,
- Herstellen einer ersten Kommunikationsverbindung (11) zwischen dem Server (1) und einer Rechenvorrichtung (2), wobei die erste Kommunikationsverbindung eine gesicherte Verbindung ist, die einen asymmetrischen Schlüsselalgorithmus verwendet,
- Herstellen einer zweiten Kommunikationsverbindung (30A, 30B) zwischen der Rechenvorrichtung (2) und der medizinischen Vorrichtung (3A, 3B),
- Abrufen, von der Rechenvorrichtung (2), des Sicherheitsschlüssels (4A, 4B) vom Server (1) über die erste Kommunikationsverbindung (11), und
- Übertragen, von der Rechenvorrichtung (2), des abgerufenen Sicherheitsschlüssels (4A, 4B) an die medizinische Vorrichtung (3A, 3B) über die zweite Kommunikationsverbindung (30A, 30B), wobei der Sicherheitsschlüssel (4A, 4B) nicht von der Rechenvorrichtung (2) gespeichert wird, sondern nur an die medizinische Vorrichtung (3A, 3B) übertragen wird, und
- nach Verteilung des dedizierten Schlüsselpaars (4A, 4B), Herstellen einer dritten Kommunikationsverbindung (31A) zwischen der medizinischen Vorrichtung (3A, 3B) und dem Server (1), um Nachrichten zwischen der medizinischen Vorrichtung (3A, 3B) und dem Server (1) unter Verwendung des Sicherheitsschlüssels (4A, 4B) zu übertragen, wobei die dritte Kommunikationsverbindung (31A) einen symmetrischen Schlüsselalgorithmus verwendet und wobei eine Nachricht unter Verwendung von mindestens einem von dem Vorrichtungsschlüssel und dem Serverschlüssel des Schlüsselpaars (4A, 4B) verschlüsselt wird.

2. Verfahren nach Anspruch 1, wobei die erste Kommunikationsverbindung (11) eine Internetprotokollverbindung ist.

3. Verfahren nach Anspruch 1 oder 2, wobei die erste Kommunikationsverbindung (11) eine HTTPS-Sitzung verwendet.

4. Verfahren nach einem der Ansprüche 1 bis 3, wobei die zweite Kommunikationsverbindung (30A, 30B) eine drahtgebundene Verbindung ist.

5. Verfahren nach einem der vorhergehenden Ansprüche, wobei die zweite Kommunikationsverbindung (30A, 30B) eine serielle Verbindung ist.

6. Verfahren nach einem der vorhergehenden Ansprüche, wobei der Server (1) eine Vielzahl von unterschiedlichen dedizierten Schlüsselpaaren (4a, 4B) generiert, die einer Vielzahl von medizinischen Vorrichtungen (3A, 3B) zugeordnet sind, wobei jedes Schlüsselpaar (4A, 4B) einen dedizierten Serverschlüssel und einen dedizierten Vorrichtungsschlüssel umfasst.

7. Verfahren nach Anspruch 6, wobei, für jede medizinische Vorrichtung (3A, 3B), das zugehörige dedizierte Schlüsselpaar (4A, 4B) vom Server (1) von der Rechenvorrichtung (2) abgerufen und an die medizinische Vorrichtung (3A, 3B) übertragen wird.

8. Verfahren nach einem der vorhergehenden Ansprüche, wobei eine Nachricht eine Signatur umfasst, die unter Verwendung von mindestens einem von dem Vorrichtungsschlüssel und dem Serverschlüssel des Schlüsselpaars (4A, 4B) generiert wird, das der medizinischen Vorrichtung (3A, 3B) und dem Server (1) zugeordnet ist.

9. Verfahren nach einem der vorhergehenden Ansprüche, wobei die medizinische Vorrichtung (1) nach der anfänglichen Verteilung des Sicherheitsschlüssels (4A, 4B) einen neuen Sicherheitsschlüssel (4A, 4B) durch Senden einer Nachricht an den Server (1) anfordert und der Server (1) den neuen Sicherheitsschlüssel (4A, 4B) in einer verschlüsselten Nachricht an die medizinische Vorrichtung (3A, 3B) sendet.

10. System zur Schlüsselverteilung, wobei das System umfasst
- einen Server (1), der konfiguriert ist, um einen Sicherheitsschlüssel (4A, 4B) in Form eines dedizierten Schlüsselpaars (4A, 4B) bereitzustellen, der für eine sichere Datenkommunikation einer medizinischen Vorrichtung (3A, 3B) verwendet wird, wobei der Server (1) konfiguriert ist, um das dedizierte Schlüsselpaar (4A, 4B) bereitzustellen, das einen Serverschlüssel und einen Vorrichtungsschlüssel umfasst,
- eine Rechenvorrichtung (2), wobei der Server (1) und die Rechenvorrichtung (2) konfiguriert sind, um eine erste Kommunikationsverbindung (11) zwischen dem Server (1) und der Rechenvorrichtung (2) herzustellen, wobei die erste Kommunikationsverbindung eine gesicherte Verbindung ist, die einen asymmetrischen Schlüsselalgorithmus verwendet,
- die medizinische Vorrichtung (3A, 3B), wobei die medizinische Vorrichtung (3A, 3B) eine Infusionsvorrichtung ist, wobei die Rechenvorrichtung (2) und die medizinische Vorrichtung (3A, 3B) konfiguriert sind, um eine zweite Kommunikationsverbindung (30A, 30B) zwischen der Rechenvorrichtung (2) und der medizinischen Vorrichtung (3A, 3B) herzustellen,
wobei die Rechenvorrichtung (2) konfiguriert ist, um den Sicherheitsschlüssel (4A, 4B) vom Server (1) über die erste Kommunikationsverbindung (11) abzurufen und den abgerufenen Sicherheitsschlüssel (4A, 4B) an die medizinische Vorrichtung (3A, 3B) über die zweite Kommunikationsverbindung (30A, 30B) zu übertragen, wobei die Rechenvorrichtung (2) ferner konfiguriert ist, um den Sicherheitsschlüssel (4A, 4B) nicht zu speichern, sondern nur den Sicherheitsschlüssel (4A, 4B) an die medizinische Vorrichtung (3A, 3B) zu übertragen, und wobei die medizinische Vorrichtung (3A, 3B) und der Server (1) konfiguriert sind, nach Verteilung des dedizierten Schlüsselpaares (4A, 4B) eine dritte Kommunikationsverbindung (31 A) zwischen der medizinischen Vorrichtung (3A, 3B) und dem Server (1) herzustellen, um Nachrichten zwischen der medizinischen Vorrichtung (3A, 3B) und dem Server (1) unter Verwendung des Sicherheitsschlüssels (4A, 4B) zu übertragen, wobei die dritte Kommunikationsverbindung (31A) einen symmetrischen Schlüsselalgorithmus verwendet und wobei eine Nachricht unter Verwendung von mindestens einem von dem Vorrichtungsschlüssel und dem Serverschlüssel des Schlüsselpaars (4A, 4B) verschlüsselt wird.

## Revendications

1. Procédé de distribution de clés entre un serveur (1) et un dispositif médical (3A, 3B), dans lequel le dispositif médical est un dispositif de perfusion, comprenant :
- la fourniture, au serveur (1), d'une clé de sécurité (4A, 4B) sous la forme d'une paire de clés dédiée (4A, 4B) à utiliser pour une communication sécurisée des données du dispositif médical (3A, 3B), dans lequel le serveur (1) fournit la paire de clés dédiée (4A, 4B) comprenant une clé de serveur et une clé de dispositif,
- l'établissement d'un premier lien de communication (11) entre le serveur (1) et un dispositif informatique (2), dans lequel le premier lien de communication est un lien sécurisé utilisant un algorithme à clé asymétrique,
- l'établissement d'un deuxième lien de communication (30A, 30B) entre le dispositif informatique (2) et le dispositif médical (3A, 3B),
- la récupération, par le dispositif informatique (2), de la clé de sécurité (4A, 4B) depuis le serveur (1) par le biais du premier lien de communication (11), et
- la transmission, par le dispositif informatique (2), de la clé de sécurité récupérée (4A, 4B) au dispositif médical (3A, 3B) par le biais du deuxième lien de communication (30A, 30B), dans lequel la clé de sécurité (4A, 4B) n'est pas stockée par le dispositif informatique (2), mais n'est transmise qu'au dispositif médical (3A, 3B), et
- lors de la distribution de la paire de clés dédiée (4A, 4B), l'établissement d'un troisième lien de communication (31A) entre le dispositif médical (3A, 3B) et le serveur (1) pour transmettre des messages entre le dispositif médical (3A, 3B) et le serveur (1) en utilisant la clé de sécurité (4A, 4B), dans lequel le troisième lien de communication (31A) utilise un algorithme de clé symétrique et dans lequel un message est crypté en utilisant au moins l'une parmi la clé de dispositif et la clé de serveur de la paire de clés (4A, 4B).

2. Procédé selon la revendication 1, dans lequel le premier lien de communication (11) est un lien de protocole Internet.

3. Procédé selon la revendication 1 ou 2, dans lequel le premier lien de communication (11) utilise une session HTTPS.

4. Procédé selon l'une des revendications 1 à 3, dans lequel le deuxième lien de communication (30A, 30B) est une liaison filaire.

5. Système selon l'une des revendications précédentes, dans lequel le deuxième lien de communication (30A, 30B) est une connexion en série.

6. Système selon l'une des revendications précédentes, dans lequel le serveur (1) génère une multiplicité de différentes paires de clés dédiées (4a, 4B) associées à une multiplicité de dispositifs médicaux (3A, 3B), chaque paire de clés (4A, 4B) comprenant une clé de serveur dédiée et une clé de dispositif dédiée.

7. Procédé selon la revendication 6, dans lequel, pour chaque dispositif médical (3A, 3B), la paire de clés dédiée associée (4A, 4B) est récupérée du serveur (1) par le dispositif informatique (2) et est transmise au dispositif médical (3A, 3B).

8. Système selon l'une des revendications précédentes, dans lequel un message comprend une signature générée en utilisant au moins l'une parmi la clé de dispositif et la clé de serveur de la paire de clés (4A, 4B) associée au dispositif médical (3A, 3B) et au serveur (1).

9. Système selon l'une des revendications précédentes, dans lequel le dispositif médical (1) demande, après la distribution initiale de la clé de sécurité (4A, 4B), une nouvelle clé de sécurité (4A, 4B) en envoyant un message au serveur (1), et le serveur (1) envoie la nouvelle clé de sécurité (4A, 4B) dans un message crypté au dispositif médical (3A, 3B).

10. Système de distribution de clés, le système comprenant
- un serveur (1) configuré pour fournir une clé de sécurité (4A, 4B) sous la forme d'une paire de clés dédiée (4A, 4B) à utiliser pour une communication sécurisée de données d'un dispositif médical (3A, 3B), dans lequel le serveur (1) est configuré pour fournir la paire de clés dédiée (4A, 4B) comprenant une clé de serveur et une clé de dispositif,
- un dispositif informatique (2), dans lequel le serveur (1) et le dispositif informatique (2) sont configurés pour établir un premier lien de communication (11) entre le serveur (1) et le dispositif informatique (2), dans lequel le premier lien de communication est un lien sécurisé utilisant un algorithme à clé asymétrique,
- le dispositif médical (3A, 3B), dans lequel le dispositif médical (3A, 3B) est un dispositif d'infusion, dans lequel le dispositif informatique (2) et le dispositif médical (3A, 3B) sont configurés pour établir un deuxième lien de communication (30A, 30B) entre le dispositif informatique (2) et le dispositif médical (3A, 3B),
dans lequel le dispositif informatique (2) est configuré pour récupérer la clé de sécurité (4A, 4B) depuis le serveur (1) par le biais du premier lien de communication (11) et pour transmettre la clé de sécurité récupérée (4A, 4B) au dispositif médical (3A, 3B) par le biais du deuxième lien de communication (30A, 30B), dans lequel le dispositif informatique (2) est en outre configuré non pas pour stocker la clé de sécurité (4A, 4B), mais pour ne transmettre que la clé de sécurité (4A, 4B) au dispositif médical (3A, 3B), et dans lequel le dispositif médical (3A, 3B) et le serveur (1) sont configurés pour établir lors de la distribution de la paire de clés dédiée (4A, 4B) un troisième lien de communication (31A) entre le dispositif médical (3A, 3B) et le serveur (1) pour transmettre des messages entre le dispositif médical (3A, 3B) et le serveur (1) en utilisant la clé de sécurité (4A, 4B), dans lequel le troisième lien de communication (31A) utilise un algorithme de clé symétrique, et dans lequel un message est crypté en utilisant au moins l'une parmi la clé de dispositif et la clé de serveur de la paire de clés (4A, 4B).
